# EUROPEAN PATENT APPLICATION

(11) **EP 2 609 912 A1**
(43) Date of publication of application: **03.07.2013**
(21) Application number: 11196239.5
(22) Date of filing: 30.12.2011
(51) Int. Cl.: A61K 9/16, A61K 9/20, A61K 9/48, A61K 31/137, A61K 31/352

(54) **Pharmaceutical combination of fingolimod and nabiximols**

(71) Applicant: Deva Holding Anonim Sirketi, 34303 Istanbul (TR)
(72) Inventor: Haas, Philipp Daniel, 34303 ISTANBUL (TR); Koc, Fikret, 34303 ISTANBUL (TR); Sivasligil, Ramazan, 34303 ISTANBUL (TR); Kandemir, Levent, 34303 ISTANBUL (TR); Ilhan, Suna Ayse, 34303 ISTANBUL (TR)

(57) **Abstract**

This invention is related to single unit formulation of fingolimod or pharmaceutically acceptable salts thereof in combination with nabiximols mostly composed of cannabidiol (CBD) type compound or derivative thereof and tetrahydrocannabinol (THC) type compound or derivative thereof in treatment of multiple sclerosis.

## Description

### Technical Field

This invention is related to single unit formulation of fingolimod or pharmaceutically acceptable salts thereof in combination with nabiximols mostly composed of cannabidiol (CBD) type compound or derivative thereof and tetrahydrocannabinol (THC) type compound or derivative thereof in treatment of multiple sclerosis.

### Background Art

Multiple sclerosis is the most common inflammatory demyelinating disease of the central nervous system (CNS) which affects more than 1.2 million people worldwide.

Multiple sclerosis (MS) is a disease in which the nerves of the central nervous system (brain and spinal cord) degenerate. Myelin provides a covering or insulation for nerves, improves the conduction of impulses along the nerves and also is important for maintaining the health of the nerves. In multiple sclerosis, inflammation causes the myelin to disappear. Consequently, the electrical impulses that travel along the nerves decelerate, that is, become slower. In addition, the nerves themselves are damaged. As more and more nerves are affected, patient suffer from a range of symptoms which affect their health related quality of life such as pain, muscle spasticity and spasm, bladder problems and sleep disturbance.

Spasticity is a common symptom of multiple sclerosis and as the disease evolves, it occurs in more than 60% of people with MS. Available treatments, for spasticity, are deemed to be partial relief and have unpleasant side effects.

Although there is no known cure for multiple sclerosis, there are few effective treatments for the symptoms of multiple sclerosis. Marketed medications are aimed to reduce the frequency of attacks or attempt to return functions after an attack or prevent new attacks. Nevertheless, it is reported that only a minority of people can benefit from current drugs and associated adverse side effects significantly limit use of these drugs.

Despite the introduction of some different medications for the treatment of multiple sclerosis over years, there is a very clear need for alternative new treatments for MS.

Fingolimod's oral multiple sclerosis therapy is a significant step for patients and helps address the unmet need for additional therapies.

Fingolimod is the first-in-class orally available drug for the treatment of relapsing multiple sclerosis. Since its market introduction, fingolimod is assessed to be promising and hopeful for several patients with regard to other medications.

Fingolimod which is a first line or a second line treatment; is indicated for the treatment of patients with relapsing forms of MS to reduce the frequency of clinical exacerbations and severity of symptoms in MS and delay the accumulation of physical.

Fingolimod-phosphate is a sphingosine 1-phosphate receptor modulator and it is metabolized by sphingosine kinase to the active metabolite, fingolimod-phosphate. Fingolimod binds with high affinity to sphingosine 1-phosphate receptors 1, 3, 4, and 5.

EP 0627406 B (YOSHITOMI PHARMACEUTICAL INDUSTRIES LTD.) 28.10.1998 firstly discloses 2-amino-1, 3-propane-diol compounds.

Fingolimod is formulated as 0.5 mg hard capsules to be taken once daily and offers patients an alternative to the currently available inject able therapies.

On the other hand, nabiximols is the first cannabis-based medicine prescribed as an add-on treatment for MS-related spasticity when people have shown inadequate response to other symptomatic treatments or found their side effects intolerable.

Nabiximols is formulated as spray for oromucosal administration with analgesic activity. The mentionned phytocannabinoid derived natural product is prepared by formulating cold organic solvent extracts of two strains of herbal cannabis sativa and includes a defined quantity of specific cannabinoids with potential analgesic activity.

The number and timing of nabiximols sprays are varying between patients, it is especially indicated in patients with moderate to severe spasticity.

Cannabis based medicinal product contains two most abundant extracts of tetrahydrocannabinol (THC), cannabidiol (CBD) both of which are known as cannabinoids.

Cannabinoids interact with G protein-coupled cannabinoid 1 (CB₁) receptors in the central nervous system, resulting in analgesic, euphoric, and anticonvulsive effects.

THC and CBD are reported to be an adjunctive treatment for symptomatic relief of neuropathic pain associated with multiple sclerosis. Each spray of nabiximols contains 2.7 mg Δ⁹-tetrahydrocannabinol (THC) and 2.5 mg cannabidiol (CBD).

Delta-9-tetrahydrocannabinol (THC) and cannabidiol(CBD) are metabolised by the cytochrome P₄₅₀ enzyme system.

Tetrahydrocannabinol (THC) also known as delta-9-tetrahydrocannabinol Δ⁹-THC or dronabinol is the main psychoactive substance found in the cannabis plant. THC appears to ease moderate pain and to be neuroprotective. The pharmacological actions of THC result from its binding to two types of G-protein coupled cannabinoid receptors; the cannabinoid receptor CB₁ which is the most abundant G protein-coupled receptor in the brain, located mainly in the central nervous system, and the CB₂ receptor, mainly present in cells of the immune system. It acts as a partial agonist on both receptors.

In pure form, THC is a glassy solid when cold, but becomes viscous and sticky if warmed. As an aromatic terpenoid, THC has a very low solubility in water, but good solubility in most organic solvents.

Since THC is very unstable to light and high temperatures, it should be protected from air during manufacturing and storage due to its extreme instability. Flammable and/or toxic gases are generated by the combination of alcohols with alkali metals, nitrides, and strong reducing agents. They react with oxoacids and carboxylic acids to form esters plus water. Oxidizing agents convert them to aldehydes or ketones.

Cannabidiol (CBD) is a major nonpsychotropic cannabinoid compound and has low affinity for CB₁ and CB₂ receptors but acts as an indirect antagonist of cannabinoid agonists.

Cannabidiol is insoluble in water but soluble in organic solvents such as ethanol, DMSO, dimethylformamide and soluble in fixed oil. At room temperature, it is a colourless crystalline solid. In strongly basic medium and the presence of air it is oxidized to a quinone. Under acidic conditions it cyclizes to THC.

### Summary of invention

The invention is directed to a pharmacutical combination of

(i) fingolimod or a pharmaceutically acceptable salt thereof

(ii) nabiximols which is mainly composed of cannabidiol (CBD) type compound or derivative thereof and tetrahydrocannabinol (THC) type compound or derivative thereof.

(iii) a pharmaceutically acceptable carrier and/or diluent or mixtures thereof.

Thus in the present invention, an oral immunosuppressant fingolimod and an analgesic nabiximols are combined in a single unit formulation or in a kit for management of multiple sclerosis symptoms. This firstly disclosed pharmaceutical combination aims to increase patient compliance in prevention disability of patients with relapsing form.

### Technical Problem

Fingolimod is an effective low dose immunosuppressant agent and the recommended dosing regimen is 0.5 mg once a day in oral administration.

For formulation scientist, pharmaceutical product development of such low-dose drug generally poses major problems. Non homogeneity of the blend, powder segregation and flowability are main challenges to overcome when formulating and manufacturing low dose drugs such as fingolimod.

Because of the quantity of drug substance is low in formulation; unacceptable content uniformity is usually encountered in tablet dosage forms.

On the other hand, nabiximols composed of cannabidiol (CBD) type compound and tetrahydrocannabinol (THC) type compound is used in treatment of neuropathic pain in patient with MS. But if neuropathic pain arises with the progression of disease, patient needs more analgesic to salve. Therefore, the phytocannabinoid derivative nabiximols's mode of use is gradual increase of dose by one spray per day until an optimum symptom relief is achieved by the patient himself. Patient usually needs up to 12 sprays per day as well as fingolimod during the course of disease.

In practice, if patients who suffer from multiple sclerosis ingest both fingolimod and nabiximols which is composed of cannabidiol (CBD) type compound or derivative thereof and tetrahydrocannabinol (THC) type compound or derivative thereof, it would be beneficiary.

But late stage multiple sclerosis' patients experience mostly difficulties in simultaneous taking of nabiximols as oromusocal spray more many times per day and once a day fingolimod. Multiple sclerosis patients suffer from common mobility limitations more than other patients.

Δ⁹⁻Tetrahydrocannabinol (THC) and cannabidiol (CBD) are known to problematic in pharmaceutical dosage forms since tetrahydrocannabinol compounds are resinous gum materials which are insoluble in water.

Thus a single unit dose formulation of low dose drug fingolimod in combination with water insoluble nabiximols is difficult to develop and may cause manufacturing problems, hinder the processibility, thereby bringing about unnecessary delays in production. As per date, combination of fingolimod in combination with water insoluble nabiximols is not developed due to presented problems.

The purpose of the present invention is to combine fingolimod and nabiximols in a single unit formulation by improving content uniformity across presented formulations, thereby increasing patient compliance in late stage MS patients. Another aim of this invention is to provide a kit containing fingolimod and nabiximols.

### Solution to Problem

In accordance with this invention, symptoms of multiple sclerosis is treated with pharmaceutically optimum amount of fingolimod in combination with with nabiximols composed of cannabidiol (CBD) type compound or derivative thereof and tetrahydrocannabinol (THC) type compound or derivative thereof.

In this invention, a fixed solid dosage form containing both sphingosine-1-phosphate receptors and phytocannabinoid based analgesic is firstly developed.

When inventors of this application attempt to develop a fixed single solid dosage form containing fingolimod or a pharmaceutically acceptable salt thereof and nabiximols which is mainly composed of cannabidiol (CBD) type compound or derivative thereof and tetrahydrocannabinol (THC) type compound or derivative thereof, many difficulties were broken out due to low dose of active pharmaceutical ingredients.

Inventors of this invention found that encountered difficulties which are related to therapeutic doses of fingolimod in combination with nabiximols can be surpassed by use of suitable excipients and by appropriate choice and optimization of manufacturing process. Selection of the correct manufacturing technique and/or equipment is mainly based on characteristics of active pharmaceutical ingredients and desired product.

Low dose of fingolimod is homogeneously distributed by geometric dilution and active ingredient coating techniques throughout the single unit dose where fingolimod is in combination with nabiximols.

Inventors of the present invention investigated the influence of drying operation in manufacturing of fingolimod and nabiximols combination. It is extremely important that the temperature and exposure time is carefully controlled due to Δ⁹-tetrahydrocannabinol (THC).

Inventors have surprisingly found that drying temperature of granules or finished dosage forms should be limited to some extent where the product temperature does not exceed 40°C during the complete manufacturing, especially drying process. As tablet press or capsule filling machine warms itself while working, the condition of 40°C should be maintained in the course of filling or pressing and or tablet press or capsule filling machine contains a cooling system.

Presented formulations surpass previous problems arising when formulating the combination in following situation; low dose of fingolimod and dose disproportionality of lipophilic of cannabinoids.

Simple and reproducible formulations and techniques are developed to minimize production site and related batch to batch manufacturing problems. Inventors preferred manufacturing techniques of direct compression and wet granulation with suitable solvent among others by taking into consideration active pharmaceutical ingredient's characteristics.

Developed formulations provide appropriate dosing for both fingolimod and nabiximols in single unit formulations by increasing patient compliance in late stage multiple sclerosis patients.

In order to increase patient adherence in the treatment of multiple sclerosis, patients benefit from swallowing only a single oral dosage combination tablet of fingolimod and nabiximols instead of a hard capsule once a day and oromuscosal sprays in different times.

### Description of embodiments

Fingolimod is indicated for the treatment of patients with relapsing forms of multiple sclerosis (MS) to reduce the frequency of clinical exacerbations and to delay the accumulation of physical disability.

On the other hand, nabiximols is indicated as add-on treatment for symptom improvement in multiple sclerosis patients with moderate to severe spasticity who have not responded adequately to other anti-spasticity medication. It is especially preferred in alleviation of neuropathic pain for MS patients.

Nabiximols which is formulated as an oromucosal spray contains a determined quantity of specific cannabinoids, with potential analgesic activity.

Their concurrent use is highly recommended and prescribed by doctors in late stage multiple sclerosis patients.

In one aspect the invention relates to pharmaceutical formulations of fingolimod or pharmaceutically acceptable salts thereof in combination with nabiximols which is mostly composed of cannabidiol (CBD) type compound or derivative thereof and tetrahydrocannabinol (THC) type compound or derivative thereof as a single unit dosage form.

A single oral dosage formulation comprising both fingolimod and nabiximols is preferred by inventors. Developed single dosage formulations will provide convenience for multiple sclerosis patients who need fingolimod and multiple doses of nabiximols together in their treatment.

Preferred embodiment of the invention is:

(i) fingolimod or a pharmaceutically acceptable salt thereof and

(ii) tetrahydrocannabinol (THC) type compound or derivative thereof and cannabinol(CBD) type compound or derivative thereof.

(iii) optionally an excipient or mixture of excipients for the manufacture of a single unit dose medicament for treatment of multiple sclerosis and alleviation of neuropathic pain related to multiple sclerosis.

In the invention, it is aimed to administer sufficient amount of nabiximols or cannabis based extracts for treatment in one single dosage form together with fingolimod or a pharmaceutically acceptable salt thereof. Administered cannabis extract may composed of tetrahydrocannabinol(THC)type compound or derivative and cannabidiol(CBD)type compound or derivative where THC: CBD ratio range is selected from 1:10 to 10:1 by weight.

According to the invention the desired dose of nabiximols containing tetrahydrocannabinol(THC)type compound or derivative and cannabidiol(CBD)type compound or derivative to combine with 0.5 mg of fingolimod or a pharmaceutically acceptable salt thereof is between 5.2 mg to 62.4 mg per day.

In the present, solid oral formulations of the invention contain an average daily dose or up to the highest permitted daily dose to be taken once a day.

Suitably, a single unit dose combination of 0.5 mg of fingolimod with 41.6 mg of nabiximols is presented for the relief of moderate pain. Besides, a appropriate formulation for combination of higher dose of 62.4 mg of nabiximols with 0.5 mg of fingolimod is presented for late stage multiple sclerosis patients.

For the manufacture of single unit dose combination of fingolimod and nabiximols, said active pharmaceutical ingredients can be separately processed, co-processed or consecutively processed. The formulations of the present invention can be produced by using mixing-granulating method, extrusion-granulating method, fluidized bed-granulating method, chopping granulating method, spray-granulating method, and crushing granulating method, one-pot processing method, direct compression, dry granulation, melt granulation, melt congealing, extrusion process or other pharmaceutically acceptable manufacturing methods

In the present invention fingolimod or pharmaceutically acceptable salt thereof can be granulated with a solution, suspension or dispersion containing, tetrahydrocannabinol(THC) type compound or derivative thereof and cannabinol(CBD) type compound or derivative thereof alone or with a surface active ingredient. Using of surface active agent is essential and compulsory for the formulations.

The following surface active agents may be used without limitation: ethylene glycol stearates, propylene glycol stearates, diethylene glycol stearates, glycerol stearates, sorbitan esters, polyhyd roxyethylenically treated sorbitan esters, phenol, PEG ethers, quaternary ammonium salts (e.g. cetyltrimethylammonium bromide), amine salts (e.g. octadecylamine hydrochloride), sodium stearate, potassium stearate, ammonium stearate, calcium stearate; triethenolamine stearate, sodium lauryl sulphate, sodium dioctylsulphosuccinate, and sodium dodecylbenzenesulphonate, phospholipids, e.g. diacylphosphatidyl glycerols, diaceylphosphatidyl cholines, diaceylphosphatidic acids, the precursors and derivatives thereof, such as for example soybean lecithin and egg yolk. Surface active agent or mixtures of surface active agents are used from 0.001 % to 7 % by weight of tablet.

Surfactants which can be used to completely or partially solubilize tetrahydrocannabinol (THC) type compound or derivative thereof and cannabinol(CBD) type compound or derivative are selected from non-ionic or ionic surfactants which are approved and safe for pharmaceutical and/or food supplements or combinations thereof. Examples of suitable surfactants, without limiting scope of the invention, are poloxamers, polyoxyethylene castor oil derivatives, polysorbates, polyoxyethylene stearates, sodium lauryl sulphate and sorbitan fatty acid esters.

Granulation can be achieved via spray granulation technique in which spraying and drying are performed simultaneously or using high or low shear granulation technique in which an additional drying step after granulation is necessary to remove solvent. Alternatively, one-pot processor, in which both granulation and drying are completed, for high or low shear granulation can be utilized.

Granulation solvent can be selected from pharmaceutically acceptable solvents or mixtures thereof. Water is preferred solvent but mixtures of water and other organic solvents can be used also. Granulation solvent can further contain additional functional excipients or cosolvents such as but not limited to binder(s), solubility enhancer(s), emulsifier(s), edible vegetable oil(s), absorption enhancer(s) or mixtures thereof.

Drying temperature is limited as such that the product temperature does not exceed 40°C during the complete manufacturing process.

Microwave and/or vacuum drying techniques is preferable for drying of wet granules containing fingolimod or a pharmaceutically acceptable salt thereof and tetrahydrocannabinol (THC) type compound or derivative thereof and cannabinol(CBD) type compound or derivative thereof.

In wet granulation, granules or powder can be filled directly into capsules or compressed into tablet cores.

Optionally, part of tetrahydrocannabinol (THC) type compound or derivative thereof and cannabinol(CBD) type compound or derivative thereof can be dry granulated optionally together with additional excipient(s) using roller compaction and/or slug tablet press technique and remaining part of tetrahydrocannabinol (THC) type compound or derivative thereof and cannabinol(CBD) type compound or derivative thereof can be granulated together with fingolimod and a pharmaceutically acceptable salt thereof

It is preferable that the product temperature dosed not exceed 40°C during compaction or slugging process.

These are prepared in a manner for example by means of dry mixing, granulating, dissolving, lyophilizing processes.

The components of a pharmaceutical combination can be dosed independently or by use of different fixed combinations. In other words administration may take place in the form of one fixed combination with two or more active ingredients, or by simultaneously administering two or more active ingredients that are formulated independently.

According to this invention, each single unit dosage form is packaged separately to protect the content from environmental effects such as light, moisture, temperature, air and handling. As cannabinoids are sensitive to light, temperature preventing light from final mixture and from tablets of at any stage of the process is of great importance due to the properties of active ingredients.

In this invention the term of "fingolimod" cover pharmaceutically acceptable bases, salts, isomers, racemates, racemic mixtures, individual diasteriomers, enantiomers. Furthermore, some of the crystalline forms for compounds of the present invention may exist as polymorphs and as such are intended to be included in the present invention. In addition, some of the compounds of the invention may form solvates with water or common organic solvents. Such solvates and hydrates, as well as anhydrous compositions, are encompassed within the scope of this invention. Preferred salt of fingolimod is hydrochloride.

In this invention the term of cannabis based extract may cover "cannabidiol" and "tetrahydrocannabinol" or pharmaceutically acceptable compounds or derivative thereof.

The term tetrahydrocannabinol or cannabidiol or other cannabinoids are further meant to encompass naturally occurring, synthetic derived or synthetically modified.

The term "composition" or "combination" covers each other and can be used instead of other one.

All forms and mixtures thereof are included within the scope of this invention.

Single unit dose pharmaceutical preparations according to the invention are, for example, tablet, bilayer tablet, tablet in tablet, capsule, tablet in capsule, inlay tablet, granules in sachet and other dosage forms suitable for oral administration.

Pharmaceutical combination may be prepared using methods as below without limiting scope of this invention;
1. Fingolimod and nabiximols are dry mixed together or,
2. Both fingolimod or nabiximols are separately granulated and then granulates are combined or,
3. One active pharmaceutical ingredient selected from fingolimod or nabiximols is granulated and then granulate is combined with other non granulated active agent or,
4. Both fingolimod and nabiximols are granulated together or,
5. One active pharmaceutical ingredient selected from fingolimod or nabiximols is dissolved alone or with one or more excipients and then other active agent alone or with one or more excipients are coated with said solution
6. One active pharmaceutical ingredient selected from fingolimod or nabiximols is dispersed alone or with one or more excipient and then other active agent alone or with one or more excipients is coated or adhered with said dispersion.
7. One active pharmaceutical ingredient selected from fingolimod or nabiximols is pressed as a tablet or tablets and then core tablet or tablets are coated with solution or suspension of the other active ingredient.

According to another aspect of the present invention, there is provided a combination of fingolimod and nabiximols which contains cannabidiol (CBD) with delta-9-tetrahydrocannabinol (THC) in a monolithic tablet formulation to use in the treatment of multiple sclerosis. Both low and high dose of different active pharmaceutical ingredients are homogenously distributed in monolithic tablet which is devoid of separate/discrete layers in the formulation.

According to another aspect of the present invention, there is provided a combination of fingolimod and nabiximols which contains cannabidiol (CBD) with delta-9-tetrahydrocannabinol (THC) in a bilayer tablet formulation in treatment of multiple sclerosis.

In another embodiment, this invention embraces bilayer tablet. Bilayer tablet comprises; a) a first layer containing fingolimod or pharmaceutically acceptable salts thereof and a pharmaceutically acceptable carrier or carriers and b) a second layer containing nabiximols or other cannabis derivatives and a pharmaceutically acceptable carrier or carriers. Alternatively, bilayer tablet comprises a) a first layer containing fingolimod or pharmaceutically acceptable salts thereof with nabiximols pharmaceutically acceptable carrier or carriers b) a second layer containing nabiximols or other cannabis derivatives and a pharmaceutically acceptable carrier or carriers.

According to another aspect of the present invention, there is provided a combination of fingolimod and nabiximols which contains nabiximols or other cannabis derivatives in inlay tablet formulation in treatment of multiple sclerosis. The inlay tablet comprises that it is encompassed by an outer tablet wherein at least a portion of one surface of the inner tablet is not surrounded by outer tablet. The inner tablet comprises fingolimod and the outer tablet comprises nabiximols or other cannabis derivatives or vice versa. An excipient or mixture of excipients can be used.

According to a another aspect of the present invention, there is provided a combination of fingolimod and nabiximols which contains cannabidiol (CBD) with delta-9-tetrahydrocannabinol (THC) or other cannabis derivatives in a tablet in tablet formulation in treatment of multiple sclerosis.

In an embodiment, the present invention provides tablet-in-tablet compositions comprising: a) a core tablet comprising: fingolimod or pharmaceutically acceptable salts thereof and a pharmaceutically acceptable carrier or carriers b) a compressed outer tablet layer comprises: nabiximols which contains cannabidiol (CBD) with delta-9-tetrahydrocannabinol (THC) or other cannabis derivatives and a pharmaceutically acceptable carrier or carriers, optionally with fingolimod. Alternatively, tablet-in-tablet compositions comprising: a) a core tablet comprising: nabiximols which contains cannabidiol (CBD) with delta-9-tetrahydrocannabinol (THC) or other cannabis derivatives and a pharmaceutically acceptable carrier or carriers optionally with fingolimod b) a compressed outer tablet layer comprising: fingolimod or pharmaceutically acceptable salts thereof and a pharmaceutically acceptable carrier or carriers

According to another aspect of the present invention, there is provided a combination of fingolimod and nabiximols which contains cannabidiol (CBD) with delta-9-tetrahydrocannabinol (THC) in a capsule formulation in treatment of multiple sclerosis.

As a yet further aspect, prepared granulates can be filled into capsules. Capsules can be consisted of separated compartments and each active ingredient and it's granulates can be filled into each compartment.

According to a another aspect of the present invention, there is provided a combination of fingolimod and nabiximols which contains cannabidiol (CBD) with delta-9-tetrahydrocannabinol (THC) or other cannabis derivatives in a capsule in capsule formulation in treatment of multiple sclerosis.

This invention embraces capsule in capsule formulations wherein smaller size capsule is encapsulated into a larger capsule. Capsule-in-capsule consists of an external capsule and internal capsule (inner capsule) located therein. It is preferred that smaller size capsule is filled with fingolimod and optionally excipients and larger capsule is filled with THC/CBD or other cannabis derivatives and optionally excipients. Capsule in capsule operations can be performed via Modu-C (HarroHofliger).

According to another aspect of the present invention a pharmaceutical dosage form contains granules or powders containing fingolimod or a pharmaceutically acceptable salt thereof in combination with nabiximols mostly composed of cannabidiol (CBD) type compound or derivative thereof and tetrahydrocannabinol (THC) type compound or derivative thereof which can be used as single dose unit package. Pharmaceutical oral dosage form can be either tablets or granules or powder in sachets

According to another aspect of the present invention, there is provided a combination of fingolimod and nabiximols which contains cannabidiol (CBD) with delta-9-tetrahydrocannabinol (THC) or other cannabis derivatives in a kit formulation in treatment of multiple sclerosis.

In accordance with this invention the kit comprising (i) therapeutically effective amount fingolimod and optionally an acceptable carrier or diluents in a unit dosage form and (ii) therapeutically effective amount of nabiximols and an acceptable carrier or diluent in a unit dosage form and (iii) a container.

According to this invention, each unit dosage form is discrete dosage forms that are packaged together. The kits of the invention can include container as separate compositions such as a divided bottle or a divided packet. Separate unit dosage forms can also be contained in undivided container. The pharmaceutical kits can include instructions as a separate sheet or optionally printed on the containers.

Based on another embodiment, this invention encompasses a kit or commercial package comprising a plurality of separate containers, wherein at least one container contains combination of fingolimod or pharmaceutically acceptable salts thereof and nabiximols or other cannabis derivatives and at least one different container contains fingolimod or nabiximols or pharmaceutically acceptable salts thereof.

The pharmaceutical combination can concurrently, separately or sequentially be administered with an instruction.

Total % quantity of drug substances may vary between 1-90% of total weight of pharmaceutical dosage form.

Preferably, total % quantity of drug substances varies between 5 - 80% of total weight of pharmaceutical dosage form.

For the present invention in combinations, fingolimod: total cannabis extract ratio range is selected from 1:1 to 1:200 by weight. Preferably range is selected from 1:10 to 1:150, more preferably range is selected from 1:10 to 1: 125. The most preferably range is 1: 125.

In pharmaceutical composition or combination, nabiximols which is composed of Δ⁹-tetrahydrocannabinol and cannabidiol or a cannabis derivative is present in an amount of from 1 mg to 150 mg, preferably in an amount of from 2 mg to 100 mg and most preferably 62.4 mg and fingolimod is present in an amount of from 0.25 mg to 1.50 mg, preferably in an amount of from 0.50 mg to 1.25 mg and most preferably 0.50 mg.

Preferred doses of combination comprises 0.5 mg fingolimod and 62.4 mg nabiximols consisting 32,4 mg delta-9-tetrahydrocannabinol (THC) and 30 mg cannabidiol (CBD) or 0.5 mg fingolimod and 41.6 mg nabiximols consisting 21.6 mg delta-9-tetrahydrocannabinol (THC) and 20 mg cannabidiol (CBD).

Total % quantity of diluent(s) may vary between 1 - 90% of total weight of pharmaceutical dosage form.

Preferably, total % quantity of diluent(s) varies between 2 - 80% of total weight of pharmaceutical dosage form.

Total % quantity of lubricant(s) may vary between 0.1 - 4% of total weight of pharmaceutical dosage form.

Preferably, total % quantity of lubricant(s) varies between 0.3 - 3% of total weight of pharmaceutical dosage form.

Numerous embodiments of the present invention are prepared by a method following manners: i) fingolimod and nabiximols are separately granulated and then they are separately compressed into mini tablets and filled into capsule altogether or, ii) fingolimod and nabiximols are separately granulated and then they are separately compressed into tablet and filled into capsule or, iii) one of the active agents, fingolimod or nabiximols, is granulated and compressed into tablet or mini-tablets and then combined with granules of other active agent, fingolimod or nabiximols, into capsule or iv) fingolimod and nabiximols are separately granulated and then directly filled into capsule without tabletting or, v) one of the active agents selected from fingolimod or nabiximols, is prepared through direct compression into tablet or mini-tablets and then combined with granules of other active agent, fingolimod or nabiximols, into capsule, or vi) fingolimod and nabiximols are granulated and filled into sachets.

According to this invention, combination includes one or more pharmaceutically acceptable excipients, carriers, or diluents. In formulations, surfactants, diluents, sweeteners, disintegrants, direct compression agents, acids for effervescent forms, binders, lubricants, glidants, colorants, alkaline effervescing agents, flavors and mixtures thereof can be used.

Diluents are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst^{™}, mixtures thereof and the like.

Glidants are, but not limited to, silicon dioxide, colloidal silicon dioxide, calcium silicate, magnesium silicate, magnesium trisilicate, talc, starch, mixtures thereof and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like .

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose,lower alkylsubstituted hydroxypropyl cellulose, polacrilin potassium, starch, pregelatinized starch, sodium alginate, mixtures thereof or whatsoever.

Direct compression agents are, but not limited to, pregelatinized starch, polyvinylpyrrolidone, methylcellulose, microcrystalline cellulose, sucrose, lactose, dextrose, sorbitol, mannitol, lactitol, xylitol, modified calcium salt, granulated corn starch, modified rice starch, compressible sugars such as Destab™, dextrates such as Emdex ^{®} dicalcium phosphate, hydroxypropylcellulose , methylcellulose , hydroxypropylmethylcellulose, polyethylene glycol, amylose,anhydrous calcium hydrogen phosphate, calcium sulphate,tribasic calcium phosphate, dibasic calcium phosphate, low-crystallinity powdered cellulose,silicified microcrystalline cellulose,chitin,chitosan hydrochloride,copovidone,croscarmellose sodium,dextrose, anhydrous lactose,anhydrous alpha lactose,anhydrous beta lactose,agglomerated lactose, spray-dried lactose, maltodextrin, mixtures thereof and the like.

Flavors are, but not limited to, cinnamon oil,essence of apple, essence of pear, essence of peach, essence of grape, essence of strawberry, essence of raspberry, essence of cherry,essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde, aldehyde C-8, aldehyde C-9, aldehyde C-12 , tolyl aldehyde, mixtures thereof and the like.

Sweeteners are but not limited to, corn syrup, dextrose, cyclamate, invert sugar, fructose, saccharin, aspartame, acesulfame-K, Stevia rebaudiana, sucralose, sorbitol, mannitol, xylitol, mixtures thereof and the like.

Pharmaceutically acceptable additives other than those described above may be included in formulations of the present invention.

The compositions may include flavour enhancers, preservatives, antiadherants, flavourings, colouring agents, surfactants, solubilizers, wetting agents and other excipients of common use. Additives ingredients will vary according to the type of compositions being manufactured and can be included in the pharmaceutical composition in any amount.

In another aspect, newly developed formulations results proved improved content uniformity.

The following examples are given for the purpose of illustration of the present invention and should not limit the scope of the invention.

### Example 1 (Monolithic Tablet Unit Formula for Direct Compression)

Fingolimod + Δ⁹-THC/CBD Monolithic Tablet:

**Table 1**

| | **mg/tablet** | **% (w/w)** |
|---|---|---|
| Fingolimod HCl | 0.5 | 0.34 |
| Δ⁹-tetrahyd rocannabin ol | 32.4 | 21.6 |
| Cannabidiol | 30.0 | 20.0 |
| Mannitol DC | 55.6 | 37.06 |
| Microcrysta lline Cellulose (Avicel PH 102) | 30.0 | 20.00 |
| Magnesium Stearate | 1.50 | 1.00 |
| **Total Core Tablet Weight** | **150.00** | **100.00** |

### Example 2 (Manufacturing Process of Example 1)

Formulation above can be prepared by means of direct compression method comprising steps of ;
1. Fingolimod is mixed with a portion of mannitol DC and microcrystalline cellulose.
2. The powder mixture is sieved by using a suitable screen and installed into container and mixed.
3. The powder prepared at step (2) is added to a portion of mannitol DC.
4. The rest of mannitol DC, Δ⁹-tetrahydrocannabinol and cannabidiol are added to the container and mixed.
5. Magnesium stearate is added and mixed.
9. Core tablets are compressed with suitable punches having average target weight of 150 mg/tablet.

### Example 3 (Monolithic Tablet Unit Formula for Spray Drying

Fingolimod + Δ⁹-THC/CBD Monolithic Tablet:

**Table 2**

| | **Mg/unit dose** | **% (w/w)** |
|---|---|---|
| Fingolimod HCl | 0.5 | 0.5 |
| Δ⁹-tetrahydrocann abinol | 32.4 | 32.4 |
| Cannabidiol | 30.0 | 30.0 |
| Crospovidone | 3.00 | 3.00 |
| Sodium Lauryl Sulfate | 1.00 | 1.00 |
| Mannitol | 24.1 | 24.1 |
| Microcrystalline Cellulose (Avicel PH 102) | 8.00 | 8.00 |
| Magnesium Stearate | 1.00 | 1.00 |
| **Total Core Tablet Weight** | **100.00** | **100.00** |

### Example 4 (Manufacturing Process of Example 3)

Formulation above can be prepared by means of wet granulation method comprising steps of ;
1. Fingolimod is dry mixed with 1/3 of mannitol.
2. The remaining part of mannitol and ½ of crospovidone are mixed with the powder mixture prepared at step(1)
3. Δ⁹-tetrahydrocannabinol, cannabidiol, is dissolved in water together with Sodium Lauryl Sulphate,
4. The solution prepared at step(3) is sprayed at powder mixture prepared at step(2) in Vector GMX Micro HSM granulator,
5. Drying the granules is performed at a temperature where the product temperature does not exceed 40°C until moisture content is less than 4% (w/w).
6. Dry granules are mixed with remaining of crospovidone and avicel PH 102.
7. Magnesium stearate is added and mixed.
8. Core tablets are compressed with suitable punches having average target weight 100 mg/tablet.

### Example 5 (Bilayer Tablet Unit Formula)

Fingolimod + Δ⁹-THC/CBD Double Layer Tablet:

**Table 3**

| | **mg/unit dose** | **% (w/w)*** |
|---|---|---|
| Δ⁹-tetrahydroca nnabinol | 32.40 | 16.20 |
| Cannabidiol | 30.00 | 15.00 |
| Poloxamer 188 | 12.00 | 6.00 |
| Mannitol DC | 12.60 | 6.30 |
| Microcrystalline Cellulose (Avicel PH 102) | 6.00 | 3.00 |
| Crospovidone | 6.00 | 3.00 |
| Magnesium Stearate | 1.00 | 0.5 |
| **Total Nabiximols Layer Weight** | **100.00** | |
| Fingolimod | 0.5 | 0.25 |
| Mannitol DC | 45.00 | 22.50 |
| PVP K-30 | 5.00 | 2.50 |
| Croscarmellose Sodium | 12.00 | 6.00 |
| Microcrystalline Cellulose (Avicel PH 302) | 36.50 | 18.25 |
| Magnesium Stearate | 1.00 | 0.50 |
| Total | 100.00 | |
| Fingolimod | | |
| Layer Weight | | |
| **Total Core** | **200.00** | |
| **Tablet Weight** | | |

* % values are calculated according to final core tablet weight.

### Example 6:(Manufacturing Process of Example 5)

Formulation above can be prepared by the method comprising steps of ;
1. For the preparation of fingolimod layer, fingolimod and a portion of mannitol are mixed and sieved through a suitable sieve.
2. Powder mixture prepared at step (1) is added to PVP and crospovidone and mixed.
3. The remaining part of mannitol and microcrystalline cellulose are added to powder mixture prepared at step (2)
4. Magnesium Stearate is added, mixed and fingolimod tablet layer is pressed.
5. For the preparation of nabiximols layer, Δ⁹-tetrahydrocannabinol, cannabidiol and poloxamer 188 are mixed and mixed through a suitable sieve.
6. Powder mixture in (5), mannitol, microcrystalline cellulose and crospovidone are mixed and mixed through a suitable sieve.
7. Magnesium stearate is added, mixed and nabiximols layer ready for compression is prepared.
8. Double layer tablets are compressed from prepared fingolimod layer and nabiximols layer using Fette 102i Tablet Press Machine.
9. Average target weight of final core tablets is 200 mg/tablet.

### Example 7(Tablet-in-tablet Unit Formula)

Fingolimod + Δ⁹-THC/CBD Tablet in Tablet:

**Table 4**

| | **mg/unit dose** | **% (w/w)*** |
|---|---|---|
| Fingolimod | 0.5 | 0.20 |
| Crospovidone | 5.0 | 2.00 |
| Mannitol DC | 21.6 | 8.64 |
| Microcystalline cellulose | 22.4 | 8.96 |
| Magnesium Stearate | 0.5 | 0.20 |
| **Total** | **50.00** | |
| **Tablet Weight** | | |
| Δ⁹-tetrah ydrocann abinol | 32.4 | 12.96 |
| Cannabidiol | 30.00 | 12.00 |
| Tween 80 | 8.00 | 3.20 |
| Mannitol | 57.6 | 23.04 |
| Crospovidone | 20.00 | 8.00 |
| Microcrystalline Cellulose (Avicel PH 101) | 50.00 | 20.00 |
| Magnesium Stearate | 2.00 | 0.80 |
| Total | 200.00 | |
| Nabiximo Is | | |
| Granules Weight | | |
| **Total** | **250.00** | |
| **Core** | | |
| **Tablet** | | |
| **Weight** | | |

* % values are calculated according to final core tablet weight

### Example 8 (Manufacturing Process of Example 7)

Formulation above can be prepared by the method comprising steps of ;
1. Fingolimod is mixed with a portion of mannitol DC and microcrystalline cellulose.
2. The powder mixture is sieved by using a suitable screen and installed into container and mixed.
3. The powder prepared at step (2) is added to a portion of mannitol DC and crospovidone.
4. Magnesium stearate is added and mixed.
5. Core tablets are compressed with suitable punches having average target weight of 50 mg/tablet.
6. Δ⁹-tetrahydrocannabinol and cannabidiol and tween 80 are dissolved in water.
7. 1/2 of crospovidone, mannitol and microcrystalline cellulose are mixed.
8. The solution prepared at step(6)is sprayed on the powder mixture prepared at step(7) in Fluid Bed Granulator (Vector VFC-30X) and dried until moisture content is less than 3% (w/w).
9. Dry granules are mixed with remaining part of crospovidone
10. Magnesium Stearate is added, mixed and
11. Using IMA S250 ZS/M Tablet Press Machine, which is capable of compressing tablet-in-tablet, fingolimod tablets are compressed with nabiximols granules.
12. Average target weight of final core tablets is 250 mg/tablet.

### Example 9 (Capsule Unit Formula)

Fingolimod + Δ⁹-THC/CBD Capsule:

**Table 5**

| | **mg/capsule** | **% (w/w)** |
|---|---|---|
| Fingolimod | 0.5 | 0.34 |
| Δ⁹-tetrahydr ocannabinol | 32.4 | 21.6 |
| Cannabidiol | 30.0 | 20.00 |
| Mannitol | 85.1 | 56.73 |
| Magnesium Stearate | 2.00 | 1.33 |
| **Total** | **150.00** | **100.00** |
| **Powder** | | |
| **Weight in** | | |
| **Capsule** | | |

### Example 10(Manufacturing Process of Example 9)

Formulation above can be prepared by the method comprising steps of ;
1. Fingolimod is mixed with a portion of mannitol.
2. Powder mixture prepared at step (1) is added to Δ⁹ -tetrahydrocannabinol, cannabidiol and the remaining part of mannitol.
3. Magnesium Stearate is added and mixed.
4. Powder mixture in Step (3) is filled into hard gelatin capsules having average target powder weight of 150 mg/capsule.

### Example 11 (Tablet in capsule Unit Formula)

Fingolimod + Δ⁹-THC/CBD Tablet Unit Formula:

**Table 6**

| | **mg/unit dose** | **% (w/w)** |
|---|---|---|
| Fingolimod | 0.5 | 0.125 |
| Δ⁹-tetrahydrocannabinol | 32.4 | 8.1 |
| Cannabidiol | 30.0 | 7.5 |
| Lactose Anhydrous | 112.1 | 28.025 |
| Pregelatinized Starch | 25.00 | 6.25 |
| Crospovidone | 40.00 | 10.00 |
| Microcrystalline Cellulose (Avicel PH 102) | 155.00 | 38.75 |
| Sodium Stearyl Fumarate | 5.00 | 1.25 |
| **Total Powder** | **400.00** | **100.00** |
| **Weight in** | | |
| **Capsule** | | |

### Example 12 (Manufacturing Process of Example 11)

Formulation above can be prepared by the method comprising steps of:
1. Lactose Anhydrous, fingolimod and a part of crospovidone are mixed and sieved through a suitable sieve.
2. Sodium Stearyl Fumarate is sieved and added.
3. Fingolimod tablets are compressed with suitable punches having average target weight of 90 mg/tablet.
4. Δ⁹-tetrahydrocannabinol, Cannabidiol, pregelatinized starch and the remaining part of crospovidone mixed.
5. Powder mixture prepared at step (4) is added to Avicel PH 102.
6. Using Zanasi 40E Capsule Filling Machine, which is capable of filling both tablet and powder into capsule, fingolimod tablets and nabiximols granules are filled into hard gelatin capsule.
7. Average target weight inside of capsules is 400 mg/capsule.

### Example 12 (Granules in sachet Unit Formula)

Fingolimod + Δ⁹-THC/CBD granules in sachet Unit Formula:

**Table 7**

| | **mg/sachet** | **% (w/w)** |
|---|---|---|
| *Fingolimod* | 0.5 | 0.1 |
| Δ⁹-tetrahydrocannabinol | 32.4 | 6.48 |
| Cannabidiol | 30.0 | 6.00 |
| Sodium Lauryl Sulfate | 16.5 | 3.3 |
| Sucralose | 30.00 | 6.0 |
| Orange Flavour | 25.00 | 5.0 |
| Mannitol | 290.6 | 58.12 |
| Crospovidone | 50.00 | 10.00 |
| Colloidal Silicon Dioxide | 5.00 | 1.00 |
| PEG 800 | 10.00 | 2.00 |
| Glycerine | 10.00 | 2.00 |
| **Total Powder** | **500.00** | **100.00** |
| **Weight in** | | |
| **Capsule** | | |

### Example 13 (Manufacturing Process of Example 12)

Pharmaceutical formulation may be prepared using methods as below without limiting scope of this invention;
1. Fingolimod, sodium lauryl sulphate, Δ⁹-tetrahydrocannabinol, cannabidiol, PEG 800 and glycerine are dissolved or dispersed in water.
2. Mannitol and ½ of crospovidone are mixed
3. Powder mixture prepared in step (2) is granulated with dispersion prepared in step (1).
4. Wet granules are dried in Collette UltimaGral 25 using both microwave and vacuuming functions until moisture content is less than 1%.
5. Dried granules are sieved through suitable sized sieve and mixed.
6. Sucralose, Orange Flavour, ½ of crospovidone are added and mixed with dry granules prepared at step (5)
6. Colloidal Silicon Dioxide and some part of dried granules are mixed and sieved through suitable sized sieve.
7. Powder mixture in step (7) is mixed with remaining dried granules and mixed.
8. Final powder mixture is filled into suitable packaging materials using sachet filling machine at average target powder weight of 500 mg/sachet.

## Claims

1. A pharmaceutical combination comprising; i) therapeutically effective amount of fingolimod or pharmaceutically acceptable salt thereof; and ii) therapeutically effective amount of nabiximols which is composed of Δ⁹-tetrahydrocannabinol and cannabidiol or a cannabis derivative and iii) a pharmaceutically acceptable carrier and/or diluent or mixtures thereof.

2. ln pharmaceutical combination, according to preceding claim, nabiximols which is composed of Δ⁹-tetrahydrocannabinol and cannabidiol or a cannabis derivative is present in an amount of from 1 mg to 150 mg, preferably in an amount of from 2 mg to 100 mg and most preferably 62.4 mg and fingolimod is present in an amount of from 0.25 mg to 1.50 mg, preferably in an amount of from 0.50 mg to 1.25 mg and most preferably 0.50 mg.

3. According to claim 1, pharmaceutical combination is prepared by a method; (i) Fingolimod and nabiximols are dry mixed together or,(ii)Both fingolimod or nabiximols are separately granulated and then granulates are combined or, (iii) One active pharmaceutical ingredient selected from fingolimod or nabiximols is granulated and then granulate is combined with other non granulated active agent or,(iv)Both fingolimod and nabiximols are granulated together or, (v)One active pharmaceutical ingredient selected from fingolimod or nabiximols is dissolved alone or with one or more excipients and then other active agent alone or with one or more excipients are coated with said solution or, (vi)One active pharmaceutical ingredient selected from fingolimod or nabiximols is dispersed alone or with one or more excipient and then other active agent alone or with one or more excipients is coated or adhered with said dispersion, (vii) One active pharmaceutical ingredient selected from fingolimod or nabiximols is pressed as a tablet or tablets and then core tablet or tablets are coated with solution or suspension of the other active ingredient.

4. According to claims of 1 to 2, pharmaceutical combination is prepared by a method : i) fingolimod and nabiximols are separately granulated and then they are separately compressed into mini tablets and filled into capsule altogether or, ii) fingolimod and nabiximols are separately granulated and then they are separately compressed into tablet and filled into capsule or, iii) one of the active agents, fingolimod or nabiximols, is granulated and compressed into tablet or mini-tablets and then combined with granules of other active agent, fingolimod or nabiximols, into capsule or iv) fingolimod and nabiximols are separately granulated and then directly filled into capsule without tabletting or, v) one of the active agents selected from fingolimod or nabiximols, is prepared through direct compression into tablet or mini-tablets and then combined with granules of other active agent, fingolimod or nabiximols, into capsule, or vi) fingolimod and nabiximols are granulated and filled into sachets.

5. According to claims of 1 to 2, pharmaceutical combination is in the form of bilayer tablet comprises a) a first layer containing fingolimod or pharmaceutically acceptable salts thereof and a pharmaceutically acceptable carrier or carriers and b) a second layer containing nabiximols or other cannabis derivatives and a pharmaceutically acceptable carrier or carriers, or a) a first layer containing fingolimod or pharmaceutically acceptable salts thereof with nabiximols pharmaceutically acceptable carrier or carriers and b) a second layer containing nabiximols or other cannabis derivatives and a pharmaceutically acceptable carrier or carriers.

6. According to claims of 1 to 2, pharmaceutical combination is in the form of inlay tablet which comprises a) an inner tablet comprising fingolimod or pharmaceutically acceptable salts thereof and a pharmaceutically acceptable carrier or carriers and b) an outer tablet comprising nabiximols thereof and a pharmaceutically acceptable carrier or carriers or,a) an inner tablet comprising nabiximols or cannabis extract based and a pharmaceutically acceptable carrier or carriers b) an outer tablet comprising fingolimod or pharmaceutically acceptable salts thereof and a pharmaceutically acceptable carrier or carriers wherein inner tablet is encompassed by an outer tablet wherein at least a portion of one surface of the inner tablet is not surrounded by outer tablet.

7. According to claims of 1 to 2, pharmaceutical combination is in the form of tablet-in-tablet comprising; a) a core tablet comprising: fingolimod or pharmaceutically acceptable salts thereof and a pharmaceutically acceptable carrier or carriers and b) a compressed outer tablet layer comprises: nabiximols and a pharmaceutically acceptable carrier or carriers, optionally with fingolimod or a) a core tablet comprising: nabiximols and a pharmaceutically acceptable carrier or carriers optionally with fingolimod and b) a compressed outer tablet layer comprising: fingolimod or pharmaceutically acceptable salts thereof and a pharmaceutically acceptable carrier or carriers.

8. Pharmaceutical combination, according to claims of 1 to 2, is in the form of capsule in capsule which consists of a) an external capsule comprising nabiximols and a pharmaceutically acceptable carrier or carriers and b) internal capsule (inner capsule) comprising fingolimod or pharmaceutically acceptable salts thereof and a pharmaceutically acceptable carrier or carriers or:a) an external capsule comprising fingolimod or pharmaceutically acceptable salts thereof and a pharmaceutically acceptable carrier or carriers and b) internal capsule (inner capsule) comprising nabiximols and a pharmaceutically acceptable carrier or carriers wherein smaller size capsule is encapsulated into a larger capsule.

9. According to claims of 1 to 2, pharmaceutical combination is in the form of kit comprising (i) therapeutically effective amount fingolimod or pharmaceutically acceptable salts thereof and optionally an acceptable carrier or diluents in a unit dosage form and (ii) therapeutically effective amount of nabiximols and an acceptable carrier or diluent in a unit dosage form and (iii) a container.

10. According to claims of 1 to 2, pharmaceutical combination is in the form of granules in sachet which comprises fingolimod or pharmaceutically acceptable salts thereof and nabiximols or other cannabis derivatives and preferably an excipient or mixtures of excipients.

11. According to preceding claims, granules or finished dosage forms are dried and/or manufactured at a temperature not exceeding 40°C.

12. According to preceding claims, combination or kit comprises 0.5 mg fingolimod and 62.4 mg nabiximols consisting 32,4 mg delta-9-tetrahydrocannabinol (THC) and 30 mg cannabidiol (CBD) or 0.5 mg fingolimod and 41.6 mg nabiximols consisting 21.6 mg delta-9-tetrahydrocannabinol (THC) and 20 mg cannabidiol (CBD).

13. According to preceding claims, formulations contain a surface active agent or mixtures of surface active agents which are used from 0.001 % to 7 % by weight of pharmaceutical formulation.
